(19) Europäisches Patentamt
European Patent Office
Office européen des brevets

(11) **EP 4 357 403 A1**

(12) **EUROPEAN PATENT APPLICATION**
published in accordance with Art. 153(4) EPC

(43) Date of publication:
**24.04.2024 Bulletin 2024/17**

(21) Application number: **22824688.0**

(22) Date of filing: **25.04.2022**

(51) International Patent Classification (IPC):
**C08L 1/02** (2006.01)     **A61K 8/73** (2006.01)
**A61Q 1/00** (2006.01)     **C08K 3/22** (2006.01)
**C08K 3/38** (2006.01)     **C08K 5/098** (2006.01)
**C08L 1/12** (2006.01)

(52) Cooperative Patent Classification (CPC):
**A61K 8/73; A61Q 1/00; C08K 3/22; C08K 3/38;
C08K 5/098; C08L 1/02; C08L 1/12**

(86) International application number:
**PCT/JP2022/018738**

(87) International publication number:
**WO 2022/264693 (22.12.2022 Gazette 2022/51)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.06.2021 JP 2021099569**

(71) Applicant: **Daicel Corporation
Osaka 530-0011 (JP)**

(72) Inventors:
• **KOBAYASHI, Keiko
Tokyo 108-8230 (JP)**
• **TAKATA, Sadaki
Higashiosaka-shi, Osaka 577-8550 (JP)**
• **OMURA, Masaya
Tokyo 108-8230 (JP)**

(74) Representative: **Grünecker Patent- und
Rechtsanwälte
PartG mbB
Leopoldstraße 4
80802 München (DE)**

(54) **COMPOSITION AND METHOD FOR PRODUCING SAME**

(57)     A composition includes particles of a cellulose derivative and a surface treatment compound. The particles of the cellulose derivative have an average particle size from 0.08 $\mu$m to 100 $\mu$m. The surface treatment compound is an organic compound having a long-chain alkyl group and/or an inorganic powder. The inorganic powder has an average particle size of 1/3 or less of the average particle size of the particles of the cellulose derivative. A cosmetic composition includes the composition. A method for producing the composition includes mixing the particles of the cellulose derivative and the surface treatment compound. In the mixing, the particles of the cellulose derivative and the surface treatment compound are dry-mixed.

2020/09/29    HL    x3.0k    30 um

FIG. 1

## Description

Technical Field

The present disclosure relates to a composition and a method for producing the composition. Specifically, the present disclosure relates to a composition including cellulose derivative particles and a method for producing the same.

Background Art

Various known fine polymer particles are blended in cosmetics for purposes such as improving the spread of the cosmetic, changing the tactile feel, imparting a wrinkle blurring effect, and improving the lubricity of a foundation or the like. Examples of such fine particles that are blended into cosmetic products include fine particles including a synthetic polymer, such as polyamide, polymethyl methacrylate (PMMA), polystyrene, polypropylene, and polyethylene. However, in recent years, due to the problem of marine pollution caused by mircoplastics, fine particles composed of non-synthetic polymer materials which have necessary properties and are environmentally friendly have been required instead of these synthetic polymers.

As a non-synthetic polymer, application of fine particles formed of a cellulose derivative derived from cellulose, which is a natural product, has been studied. For example, Patent Document 1 describes cellulose acetate particles having an average particle size from 80 nm to 100 $\mu$m, a sphericity from 0.7 to 1.0, a degree of surface smoothness from 80% to 100%, and a total substitution degree of acetyl from 0.7 to 2.9.

Patent Document 2 discloses cellulose derivative particles including an alkoxy group having 2 or more carbon atoms or an acyl group having 3 or more carbon atoms, wherein the cellulose derivative particles have an average particle size from 80 nm to 100 $\mu$m, a sphericity from 70% to 100%, a degree of surface smoothness from 80% to 100%, and a total substitution degree from 0.7 to 3.

Patent Document 3 describes cellulose acetate particles having an average particle size from 80 nm to 100 $\mu$m, a sphericity from 0.7 to 1.0, a degree of surface smoothness from 80% to 100%, a surface contact angle of 100° or more, and a total substitution degree of acetyl from 0.7 to 2.9.

Citation List

Patent Documents

Patent Document 1: JP 6609726 B
Patent Document 2: JP 2020-152851 A
Patent Document 3: JP 6694559 B

Summary of Invention

Technical Problem

With the fine particles disclosed in Patent Documents 1 to 3, good tactile feel suitable for a cosmetic composition and a light scattering (soft focus) effect can be provided. However, these effects largely depend on the particle shape such as sphericity and degree of surface smoothness. In addition, in the cosmetic composition, various solvents may be used depending on the application, and the blending of a formulation may be changed. Polymer fine particles to be blended in cosmetic compositions are required to have high dispersibility especially in hydrophobic solvents and formulations. There is still room for improvement in the particle dispersibility of known fine particles.

An object of the present disclosure is to provide a composition that has good tactile feel and is excellent in particle dispersibility regardless of the particle shape, and a method for producing the composition.

Solution to Problem

The composition according to the present disclosure includes particles of a cellulose derivative and a surface treatment compound. The particles of the cellulose derivative have an average particle size from 0.08 $\mu$m to 100 $\mu$m. The surface treatment compound is an organic compound having a long-chain alkyl group and/or an inorganic powder. The inorganic powder has an average particle size of 1/3 or less of the average particle size of the particles of the cellulose derivative.

Preferably, the organic compound has a long-chain alkyl group having from 8 to 22 carbon atoms. Preferably, the organic compound is a compound selected from the group consisting of a cationic surfactant and a metal soap, or Nε-lauroyl-L-lysine.

Preferably, the surface treatment compound is one or two or more selected from the group consisting of zinc stearate, Nε-lauroyl-L-lysine, boron nitride, and zinc oxide. A content of the surface treatment compound may be from 3.0 wt.% to 10.0 wt.%.

Preferably, the cellulose derivative has an alkoxyl group having from 2 to 20 carbon atoms or an acyl group having from 2 to 40 carbon atoms. Preferably, the cellulose derivative is cellulose acylate. Preferably, the cellulose derivative is cellulose acetate. A total substitution degree of the cellulose derivative is from 2.0 to 3.2.

The particles of the cellulose derivative may have a sphericity from 0.7 to 1.0 and a degree of surface smoothness from 80% to 100%.

A cosmetic composition of the present disclosure includes the composition described in any one of the above.

A method for producing the composition according to the present disclosure includes mixing particles of a cellulose derivative having an average particle size from 0.08 μm to 100 μm and a surface treatment compound, wherein the particles of the cellulose derivative and the surface treatment compound are dry-mixed. The surface treatment compound is an organic compound having a long-chain alkyl group and/or an inorganic powder. The inorganic powder has an average particle size of 1/3 or less of the average particle size of the particles of the cellulose derivative.

The method for producing the composition may further include pulverizing the surface treatment compound before the mixing.

Advantageous Effects of Invention

The composition of the present disclosure, including, as a surface treatment compound, an organic compound having a long-chain alkyl group and/or an inorganic powder having an average particle size of 1/3 of the average particle size of the particles of the cellulose derivative, enables good tactile feel and achieves high particle dispersibility in various solvents and formulations to be used in cosmetic compositions.

Brief Description of Drawings

FIG. 1 is an SEM image of a composition according to an embodiment of the present disclosure.
FIG. 2 is an SEM image of a composition according to another embodiment of the present disclosure.
FIG. 3 is an image for explaining a method for evaluating a degree of surface smoothness (%).
FIG. 4 is an image for explaining a method for evaluating a degree of surface smoothness (%).

Description of Embodiments

Hereinafter, an example of a preferred embodiment will be specifically described. Note that each of the configurations, combinations thereof, and the like in each of the embodiments are an example, and various additions, omissions, substitutions, and other changes may be made as appropriate without departing from the spirit of the present disclosure. The present disclosure is not limited by the embodiments and is limited only by the claims. Each aspect disclosed in the present specification can be combined with any other feature disclosed herein.

In the present specification, "from X to Y" indicating a range means "X or more and Y or less". Unless otherwise noted, all test temperatures are room temperature (20°C ± 5°C).

Composition

The composition of the present disclosure includes particles of a cellulose derivative and a surface treatment compound. The particles of the cellulose derivative have an average particle size from 0.08 μm to 100 μm. The surface treatment compound is an organic compound having a long-chain alkyl group and/or an inorganic powder. The inorganic powder has an average particle size of 1/3 or less of the average particle size of the particles of the cellulose derivative. In this composition, the surface treatment compound is present on a surface of and/or between the cellulose derivative particles having a specific average particle size.

With the surface treatment compound present on the particle surface, the cellulose derivative particles can have surface physical properties suitable for solvents and formulations to be used in cosmetic compositions. The composition achieves high particle dispersibility in a variety of solvents and formulations and enables good tactile feel. In addition, the surface treatment compound present between the particles further improves the tactile feel of the cellulose derivative particles.

Here, the "composition" means a mixture including two or more compounds. In particular, a composition including cellulose derivative particles and a surface treatment compound is referred to as "composition of the present disclosure". The composition of the present disclosure is substantially a solid composition, preferably a powder. As long as the effect of the present disclosure is obtained, the surface treatment compound and the cellulose derivative particles may be in a physically attached state or in a chemically bonded state. The composition of the present disclosure may contain a substance not specified in the present specification in addition to the cellulose derivative particles and the surface treatment compound as long as the effect of the present disclosure is not inhibited.

Surface Treatment Compound

The surface treatment compound in the composition of the present disclosure is an organic compound having a long-chain alkyl group and/or an inorganic powder. The inorganic powder has an average particle size of 1/3 or less of the average particle size of the particles of the cellulose derivative. The surface treatment compound suitable for the present disclosure may be a material that is substantially insoluble in aqueous and non-aqueous solvents. Here, "insoluble" means that the solubility in a solvent at 25°C is less than 0.1%.

The type of the organic compound having a long-chain alkyl group is not particularly limited, but the number of carbon atoms of the long-chain alkyl group is preferably 5 or more, more preferably 7 or more, and still more preferably 8 or more, from the viewpoint of improving dispersibility in a solvent or a formulation to be used in a cosmetic composition. From the viewpoint of easy mixing with the cellulose derivative particles, the number of carbon atoms of the long-chain alkyl group is preferably 22 or less. The number of carbon atoms of the long-chain alkyl group may be from 5 to 22, from 7 to 22, or from 8 to 22. Two or more organic compounds having different alkyl groups may be used in combination.

The organic compound having a long-chain alkyl group is preferably a powder from the viewpoint of ease of use in the production method described later. The shape of the particles constituting the powder is not particularly limited, and may be any of a spherical shape, a plate-like shape, a needle-like shape, and an irregular shape.

In the present disclosure, the organic compound having a long-chain alkyl group may be an amino acid derivative. The amino acid derivative is preferably N$\varepsilon$-lauroyl-L-lysine.

In the present disclosure, the organic compound having a long-chain alkyl group may be a cationic surfactant. Examples of the cationic surfactant include a quaternary ammonium salt and/or an amine salt. A quaternary ammonium salt represented by the following Formula (1) is preferable from the viewpoint of improving the tactile feel and the particle dispersibility.

$$R^1R^2R^3R^4N^+X^- \qquad (1)$$

In Formula (1), $X^-$ is a halogen ion, $R^1$, $R^2$, $R^3$, and $R^4$ are each independently an alkyl group having from 1 to 25 carbon atoms which may have a substituent, and at least one of $R^1$, $R^2$, $R^3$, and $R^4$ is an alkyl group having 12 or more carbon atoms.

Specific examples of the cationic surfactant represented by Formula (1) include stearyltrimethylammonium chloride, distearyldimethylammonium chloride, behenyltrimethylammonium chloride, distearyldiethylammonium chloride, decyltriethylammonium chloride, decyl dimethyl hydroxyethyl ammonium chloride, coconut oil trimethyl ammonium chloride, coconut oil methyl dihydroxy ethyl ammonium chloride, myristyltrimethylammonium chloride, lauryltrimethylammonium chloride, distearyldimethylammonium bromide, and stearyltrimethylammonium bromide.

Further, in the present disclosure, the organic compound having a long-chain alkyl group may be a metal soap. The metal soap is defined as a non-alkali metal salt of a higher fatty acid. As the higher fatty acid, a fatty acid having from 12 to 25 carbon atoms, which may be a saturated fatty acid or an unsaturated fatty acid. Examples of such a fatty acid soap include zinc salts, calcium salts, magnesium salts, and aluminum salts of lauric acid, myristic acid, palmitic acid, stearic acid, behenic acid, oleic acid, linoleic acid, $\alpha$-linolenic acid, erucic acid, and the like. An alkaline earth metal salt of a fatty acid having from 12 to 25 carbon atoms is preferable, a zinc salt is more preferable, and zinc stearate is particularly preferable.

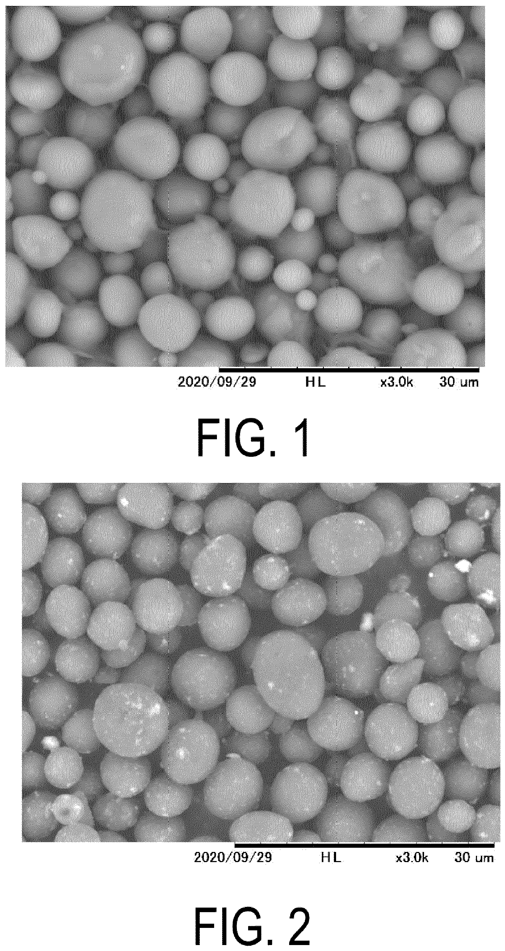

FIG. 1 is a scanning electron micrograph (3000 times) of a composition including an organic compound having a long-chain alkyl group as the surface treatment compound. From FIG. 1, it is presumed that in this composition, the organic compound having a long-chain alkyl group adheres to the surface of the cellulose derivative particles.

In the composition of the present disclosure, the average particle size of the inorganic powder is 1/3 or less of the average particle size of the cellulose derivative particles. From the viewpoint of obtaining good tactile feel and high particle dispersibility, the average particle size of the inorganic powder is more preferably 1/10 or less of the average particle size of the cellulose derivative particles. The average particle size of the inorganic powder is a volume-based median diameter, and is measured in the same manner as the average particle size of the cellulose derivative particles described later.

The average particle size of the inorganic powder is not particularly limited as long as it is 1/3 or less of the average particle size of the cellulose derivative particles, but is preferably 5 $\mu$m or less, and more preferably 2 $\mu$m or less, from the viewpoint of obtaining excellent tactile feel. The average particle size of the inorganic powder is preferably 0.01 $\mu$m or more, more preferably 0.08 $\mu$m or more, and still more preferably 0.1 $\mu$m or more, from the viewpoint of easy mixing with the cellulose derivative particles.

The particle shape of the inorganic powder is not particularly limited as long as the effect of the present disclosure is achieved, and may be, for example, any of a spherical shape, a plate-like shape, a needle-like shape, a granular shape, and an irregular shape. Preferable examples of the inorganic powder include zinc oxide, titanium oxide, magnesium oxide, boron nitride, silicon nitride, barium sulfate, calcium sulfate, magnesium sulfate, calcium carbonate, magnesium carbonate, talc, mica, kaolin, sericite, mica, vermiculite, hydilite, bentonite, montmorillonite, hectorite, kaolinite, zeolite, ceramic powder, hydroxyapatite, calcium phosphate, silicic acid, aluminum silicate, magnesium silicate, aluminum magnesium silicate, and calcium silicate. Boron nitride and zinc oxide are preferable, and zinc oxide having a large specific surface area is more preferable from the viewpoint of easy mixing with the cellulose derivative particles and obtaining good tactile feel.

FIG. 2 is a scanning electron micrograph (3000 times) of a composition including an inorganic powder having an average particle size of 1/3 or less of the average particle size of the cellulose derivative particles as the surface treatment compound. In FIG. 2, the white portion indicates the inorganic powder. As in the figure, in this composition, some of the inorganic powder is present on the surface of the cellulose derivative particles, and some of the inorganic powder is present as an aggregate between the particles.

The surface treatment compound contained in the composition of the present disclosure may be one type or two or more types. From the viewpoint of easily obtaining good tactile feel and high particle dispersibility, the surface treatment compound is preferably a compound selected from the group consisting of a cationic surfactant and a metal soap, or a surface treatment compound that is N$\varepsilon$-lauroyl-L-lysine, more preferably one or two or more selected from the group consisting of zinc stearate, N$\varepsilon$-lauroyl-L-lysine, boron nitride, and zinc oxide, and still more preferably one or two or more selected from the group consisting of zinc stearate, N$\varepsilon$-lauroyl-L-lysine, boron nitride, and zinc oxide in the form of fine particles.

From the viewpoint of obtaining surface physical properties suitable for blending into a cosmetic composition, the content of the surface treatment compound in the composition of the present disclosure is preferably 1.0 wt.% or greater, more preferably 3.0 wt.% or greater, and particularly preferably 5.0 wt.% or greater. From the viewpoint that the physical properties of the cellulose derivative particles are not inhibited, the content of the surface treatment compound is preferably 50.0 wt.% or less, more preferably 30.0 mass% or less, and particularly preferably 10.0 wt.% or less. The content of the surface treatment compound may be from 1.0 wt.% to 50.0 wt.%, from 1.0 wt.% to 30.0 wt.%, from 1.0 wt.% to 10.0 wt.%, from 3.0 wt.% to 50.0 wt.%, from 3.0 wt.% to 30.0 wt.%, from 3.0 wt.% to 10.0 wt.%, from 5.0 wt.% to 50.0 wt.%, from 5.0 wt.% to 30.0 wt.%, or from 5.0 wt.% to 10.0 wt.%. When two or more surface treatment compounds are used in combination, the total content thereof preferably satisfies the above ranges.

Cellulose Derivative Particles

The average particle size of the cellulose derivative particles may be from 0.08 $\mu$m to 100 $\mu$m, 0.1 $\mu$m or more, 1 $\mu$m or more, 2 $\mu$m or more, or 4 $\mu$m or more. In addition, the average particle size may be 80 $\mu$m or less, 40 $\mu$m or less, 20 $\mu$m or less, or 14 $\mu$m or less. When the average particle size is 100 $\mu$m or less, the tactile feel and the light scattering (soft focus) effect further improve. Cellulose derivative particles having an average particle size of 0.08 $\mu$m or more can be easily produced. Further, examples of the tactile sensation include skin feel and tactile sensation of a cosmetic composition containing the cellulose derivative particles, in addition to tactile sensation in directly touching the cellulose derivative particles.

The average particle size can be measured using dynamic light scattering. The average particle size is measured specifically as follows. First, a sample is prepared by adding the cellulose derivative particles to pure water to make a concentration of 100 ppm, and forming a pure water suspension using an ultrasonic vibrating device. Subsequently, the volume-frequency particle size distribution is measured by laser diffraction (with "Laser Diffraction/Scattering Particle Size Distribution Measuring Device LA-960" available from Horiba Ltd., ultrasonic treatment for 15 minutes, refractive index (1.500, medium (water; 1.333)). The particle size corresponding to 50% of the integrated scattering intensity in the volume-frequency particle size distribution is determined as the average particle size. That is, the average particle size ($\mu$m) in the present specification is a volume-based median diameter.

The coefficient of particle size variation of the cellulose derivative particles may be from 0% to 60%, or from 2% to 50%. The coefficient of the particle size variation (%) can be calculated by an equation: (standard deviation of particle size)/(average particle size) x 100.

The sphericity of the cellulose derivative particles is preferably from 0.7 to 1.0, more preferably from 0.8 to 1.0, and still more preferably from 0.9 to 1.0. Cellulose acylate particles with a sphericity of 0.7 or more can provide a better tactile feel, and, for example, a cosmetic composition containing such particles has further improved skin feeling and soft-focus effect.

The sphericity can be measured by the following method. From an image of particles observed with a scanning electron microscope (SEM), 30 particles are randomly selected, the major axis length and the minor axis length of each of the randomly selected particles are measured, the (minor axis length)/(major axis length) ratio of each particle is determined, and then the average value of the (minor axis length)/(major axis length) ratios for the randomly selected particles is defined as the sphericity. The particles can be determined to be closer to a true sphere as the sphericity approaches 1.

The degree of surface smoothness of the cellulose acylate particles is preferably from 80% to 100%, more preferably from 85% to 100%, and still more preferably from 90% to 100%. When the degree of surface smoothness is 80% or greater, a better tactile feel is achieved. The tactile feel becomes more preferable as the degree of surface smoothness becomes closer to 100%.

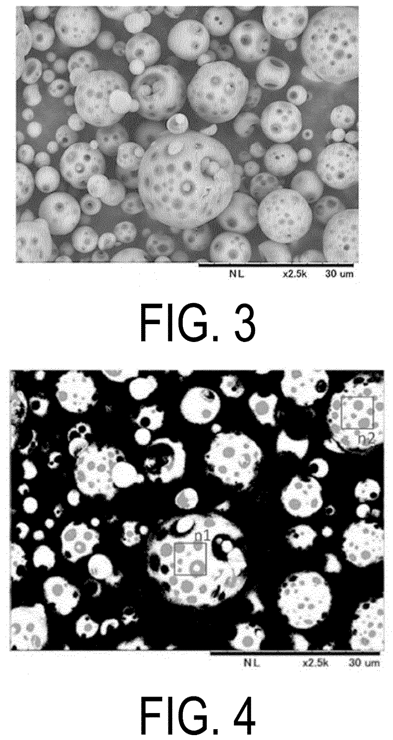

Hereinafter, a method for measuring the degree of surface smoothness will be described with reference to FIGS. 3 and 4 using sample particles having a low degree of surface smoothness. The degree of surface smoothness can be determined by taking a scanning electron micrograph of the particles, observing recesses and protrusions of the particle surfaces, and determining the degree of surface smoothness on the basis of the area of recessed portions on the surfaces. Specifically, a scanning electron micrograph of the particles is taken at a magnification of 2500 to 5000 times (see FIG. 3 for an example of the micrograph of the particles), and the image is binarized using an image processing device WinROOF (available from Mitani Corporation) (See FIG. 4 for the binarized image of the micrograph of FIG. 3). The area ratio of a portion (shade portion) corresponding to a recess of recesses and protrusions in a region smaller than a particle, the region including the center and/or near the center of one particle (for example, see the regions n1 and n2 in FIG. 3), is calculated, and the degree of surface smoothness (%) of the one particle is calculated with the following formula: The size of the region may be 5 $\mu$m square when the particle size is 15 $\mu$m.

Degree of surface smoothness of one particle (%) = (1 - area ratio of recesses) $\times$ 100

Area ratio of recesses = area of recessed portions in the any area/the any area

The average value of the degree of surface smoothness of randomly selected 10 particle samples, that is, n1 to n10, is taken as the degree of surface smoothness (%) in the present disclosure. The higher this numerical value, the higher the degree of surface smoothness is.

The cellulose derivative forming the cellulose derivative particles of the present disclosure preferably includes an alkoxy group having from 2 to 20 carbon atoms or an acyl group having from 2 to 40 carbon atoms. The cellulose derivative particles may include both an alkoxy group having from 2 to 20 carbon atoms and an acyl group having from 2 to 40 carbon atoms.

The number of carbon atoms of the alkoxy group of the cellulose derivative may be 3 or more, or 5 or more. The number of carbon atoms of the alkoxy group may be 18 or less, and preferably 8 or less. The cellulose derivative may include two or more types of alkoxy groups having different numbers of carbon atoms. The cellulose derivative may include both an alkoxy group having from 2 to 20 carbon atoms and an alkoxy group having 1 carbon (a methoxy group).

Examples of the alkoxy group having from 2 to 20 carbon atoms include an ethoxy group, a protoxy group, a butoxy group, a pentoxy group, a hexoxy group, a heptoxy group, and an octoxy group. These alkoxy groups may have a substituent as long as the effect of the present disclosure is obtained.

The number of carbon atoms of the acyl group of the cellulose derivative may be 3 or more, or 4 or more. The number of carbon atoms of the acyl group may be 30 or less, or may be 20 or less. The cellulose derivative may include two or more types of acyl groups having different numbers of carbon atoms.

Examples of the acyl group having from 2 to 40 carbon atoms include an acetyl group, a propionyl group, a butyryl group, a pentanoyl (valeryl) group, a hexanoyl group, a heptanoyl group, an octanoyl group, a nonanoyl group, an undecanoyl group, a dodecanoyl group, a tridecanoyl group, a tetradecanoyl (myristoyl) group, a pentadecanoyl group, a hexadecanoyl group, a heptadecanoyl group, and an octadecanoyl (stearoyl) group. These acyl groups may have a substituent as long as the effect of the present disclosure is obtained.

A cellulose derivative having an acyl group having from 2 to 40 carbon atoms (i.e., cellulose acylate) is preferable from the viewpoint of obtaining good tactile feel and particle dispersibility suitable for a cosmetic composition. The acyl group is preferably one or two or more selected from the group consisting of an acetyl group, a propionyl group, and a butyryl group. The acyl group is more preferably an acetyl group. The cellulose derivative of the present disclosure may be cellulose acetate.

The total substitution degree of the cellulose derivative of the cellulose derivative particles may be from 0.7 to 3.2, from 1.0 to 3.2, from 1.4 to 3.1, or from 2.0 to 3.0. This is because it is easy to produce particles having excellent moldability and good tactile feel.

The total substitution degree of the cellulose derivative can be measured by the following method. First, the total substitution degree of the cellulose derivative is the sum of each substitution degree at the 2-, 3-, and 6-positions of the glucose ring of the cellulose derivative, and each substitution degree at the 2-, 3-, and 6-positions of the glucose ring of the cellulose derivative can be measured by NMR according to the method of Tezuka (Tezuka, Carbonydr. Res. 273, 83 (1995)). That is, the free hydroxyl group of the cellulose derivative is acylated with a carboxylic anhydride in pyridine. The type of the carboxylic anhydride used here should be selected according to the purpose of the analysis; for example, when the degree of propionyl substitution of cellulose acetate propionate is analyzed, acetic anhydride is suitable, and when the degree of acetyl substitution is analyzed, propionic anhydride is suitable. The solvent and the acid anhydride of the acylation reaction may be appropriately selected according to the cellulose derivative to be analyzed.

A sample produced by acylation is dissolved in deuteriochloroform and the $^{13}$C-NMR spectrum is measured. For example, when the substituent is an acetyl group, a propionyl group, or a butyryl group, the carbon signals of the acetyl group appear in the region from 169 ppm to 171 ppm in the order of the 2-, 3-, and 6-positions from the high magnetic field; the carbonyl carbon signals of the propionyl group appear in the region from 172 ppm to 174 ppm in the same order; and the carbon signals of the butyryl group appear likewise in the region from 171 ppm to 173 ppm in the order of the 2-, 3-, and 6-positions from the high magnetic field side. In another example, when a cellulose derivative having a propionyl group is analyzed, or when a cellulose derivative having no propionyl group is treated with propionic anhydride and then the propionyl substitution degree is analyzed, carbonyl carbon signals of the propionyl group appear in the same order in a region from 172 ppm to 174 ppm.

The total substitution degree of the cellulose derivative treated with the anhydrous carboxylic acid by the method of Tezuka or a method similar thereto is 3.0, and thus, each acyl substitution degree at the 2-, 3-, and 6-positions of the glucose ring in the original cellulose derivative can be determined by normalizing a total sum of the areas of the carbonyl carbon signal of the acyl group originally included in the cellulose derivative and the carbonyl signal of the acyl group introduced by the carboxylic anhydride treatment to 3.0, and determining the presence ratio of the acetyl group and the propionyl group at each corresponding position (area ratio of each signal). It goes without saying, a substituent containing an acyl group that can be analyzed by this method is only a substituent group that does not correspond to the carboxylic anhydride used in the treatment for an analytical purpose.

However, if it is known in advance that the total substitution degree of the 2-, 3-, and 6-positions of the glucose ring of the cellulose derivative of a sample is 3.0, and all the substituents thereof are limited to substituents, such as an acetyl group and a propionyl group, the NMR spectrum can be measured by dissolving the sample directly in deuteriochloroform without acylation. If all the substituents are an acetyl group and a propionyl group, the carbon signals of the acetyl group appears in the region from 169 ppm to 171 ppm in the order of 2-, 3-, and 6-positions from high magnetic field, and the carbon signals of the propionyl group appears in the region from 172 ppm to 174 ppm in the same order, as in the case including acylation, and thus the substitution degree, such as each of acetyl and propionyl substitution degrees at the 2-, 3-, and 6-positions of the glucose ring in the cellulose derivative, can be determined from the presence ratio of the acetyl group and the propionyl group at each corresponding position (in other words, the area ratio of each signal).

The cellulose derivative particles of the present disclosure may contain a plasticizer but does not have to contain a plasticizer. In the present disclosure, the plasticizer refers to a compound capable of increasing the plasticity of the cellulose derivative. The plasticizer is not particularly limited, and examples thereof include an adipic acid-based plasticizer, a citric acid-based plasticizer, a glutaric acid-based plasticizer, succinic acid-based plasticizers, sebacic acid-based plasticizers, glycerin ester-based plasticizers, neopentyl glycol, and phosphoric acid-based plasticizers. The cellulose derivative particles may contain two or more plasticizers. One or two or more selected from the group consisting of acetyl triethyl citrate, triacetin, and diacetin are preferable.

When the cellulose derivative particles contain a plasticizer, the content of the plasticizer contained in the cellulose derivative particles is not particularly limited. For example, in relation to the weight of the cellulose derivative particles, the content of the plasticizer may be greater than 0 wt.% and less than or equal to 40 wt.%, from 2 wt.% to 40 wt.%, from 10 wt.% to 30 wt.%, or from 15 wt.% to 20 wt.%.

The content of the plasticizer in the cellulose derivative particles is determined by dissolving the cellulose derivative particles in a solvent capable of dissolving the cellulose derivative particles and measuring the solvent with $^{1}$H-NMR.

Cosmetic Composition

The composition of the present disclosure is excellent in tactile feel, well dispersed in various solvents or formulations, and thus can be suitably used in cosmetic compositions. The cosmetic composition including the composition of the present disclosure fills in and smooth out the recesses and protrusions of the skin and scatters light in various directions, and thus provides an effect of making wrinkles and the like less noticeable (soft-focus effect).

Examples of the cosmetic composition include foundations, such as liquid foundations and powder foundations; concealers; sunscreens; makeup bases; lipsticks and lipstick bases; Oshiroi powders, such as body powders, solid white powders, and face powders; solid powder eye shadows; wrinkle masking creams; and skin and hair external produces used mainly for cosmetic purposes, such as skin care lotions, and the dosage form is not limited. The dosage form may be any of a liquid preparation, such as an aqueous solution, a milky lotion, and a suspension; a semi-solid preparation, such as a gel and a cream; or a solid preparation, such as a powder, a granule, and a solid. In addition, the dosage form may be an emulsion preparation, such as a cream and a milky lotion; an oil gel preparation, such as a lipstick; a powder preparation, such as a foundation; an aerosol preparation, such as a hair styling agent; or the like.

Method for Producing Composition

A method for producing the composition according to the present disclosure includes mixing particles of a cellulose derivative having an average particle size of from 0.08 $\mu$m to 100 $\mu$m and a surface treatment compound. In the mixing, the particles of the cellulose derivative and the surface treatment compound are dry-mixed. The surface treatment compound is an organic compound having a long-chain alkyl group and/or an inorganic powder. The average particle size of the inorganic powder is 1/3 or less of the average particle size of the cellulose derivative particles. According to this production method, desired surface physical properties can be efficiently imparted to the cellulose derivative particles without using water, an organic solvent, or the like. The composition produced by this production method has good tactile feel and is excellent in dispersibility in a solvent and a formulation to be used in a cosmetic composition. As necessary, the production method may include drying a mixture of the cellulose derivative particles and the surface treatment compound produced in the mixing.

In the present disclosure, dry-mixing means mixing without adding a liquid component other than the liquid component contained in the mixture to be subjected to the mixing. In the production method of the present disclosure, the solid content concentration of the material to be mixed containing the cellulose derivative particles and the surface treatment compound may be 90 wt.% or greater, 95 wt.% or greater, or 98 wt.% or greater.

As long as the effect of the present disclosure is achieved, the dry mixing method is not particularly limited, and a known mixing means is appropriately selected and used. Examples thereof include mixing devices such as a ball mill, a sand mill, a bead mill, a homogenizer, a planetary mixer, and a filmix. The order of mixing the cellulose derivative particles and the surface treatment compound is not particularly limited. The cellulose derivative particles and the surface treatment compound weighed at a predetermined ratio may be simultaneously charged into a mixing device, or a predetermined amount of the surface treatment compound may be charged into a mixing device and stirred, and then the cellulose derivative particles may be charged and mixed.

The mixing conditions are appropriately adjusted depending on the type and amount of each component and the type of the mixer. The mixing temperature may be a temperature at which the surface treatment compound and the cellulose derivative are not thermally deteriorated. For example, the mixing temperature may be 50°C or below, 40°C or less, or 30°C or less. From the viewpoint of energy saving and cost reduction, mixing at room temperature is preferable. The mixing time may be appropriately adjusted depending on the production scale, the mixing temperature, and the like.

Specific examples of the organic compound having a long-chain alkyl group and the inorganic powder having an average particle size of 1/3 or less of the average particle size of the cellulose derivative particles as the surface treatment compound are as described above. One or two or more surface treatment compounds selected from the group consisting of zinc stearate, N$\varepsilon$-lauroyl-L-lysine, boron nitride, and zinc oxide are preferable, and one or two or more surface treatment compounds selected from the group consisting of zinc stearate, N$\varepsilon$-lauroyl-L-lysine, boron nitride, and zinc oxide in the form of dine particles are more preferable.

In the production method of the present disclosure, the addition amount of the surface treatment compound is not particularly limited, and is appropriately adjusted according to the physical properties of the cellulose derivative particles, the type of the surface treatment compound, and the like.

From the viewpoint of improving the particle dispersibility, the addition amount of the surface treatment compound is preferably 1.0 wt.% or greater, more preferably 3.0 wt.% or greater, particularly preferably 5.0 wt.% or greater with respect to the cellulose derivative particles. From the viewpoint of reducing aggregation, the addition amount of the surface treatment compound is preferably 50.0 wt.% or less, more preferably 30.0 wt.% or less, and particularly preferably 10.0 wt.% or less. The addition amount of the surface treatment compound may be from 1.0 wt.% to 50.0 wt.%, from 1.0 wt.% to 30.0 wt.%, from 1.0 wt.% to 10.0 wt.%, from 3.0 wt.% to 50.0 wt.%, from 3.0 wt.% to 30.0 wt.%, from 3.0 wt.% to 10.0 wt.%, from 5.0 wt.% to 50.0 wt.%, from 5.0 wt.% to 30.0 wt.%, or from 5.0 wt.% to 10.0 wt.%. When two or more surface treatment compounds are used in combination, the total addition amount thereof preferably satisfies the above ranges.

As necessary, the production method of the present disclosure may further include pulverizing the surface treatment compound before the mixing. In the pulverizing, the surface treatment compound is preferably pulverized so that the average particle size of the resulting ground product is smaller than the average particle size of the cellulose derivative particles. Through this pulverizing, the surface treatment compound and the cellulose derivative particles are more uniformly mixed, and the particle dispersibility of the resulting composition improves. In addition, it is possible to avoid a decrease in tactile feel due to inclusion of the surface treatment compound in a lump form.

A means for pulverizing the surface treatment compound is not particularly limited, and a known pulverizing means may be used. The surface treatment compound may be pulverized using the mixing device used in the mixing. The pulverizing conditions are adjusted so that the average particle size of the resulting ground product is 1/3 or less, preferably 1/10 or less, of the average particle size of the cellulose derivative particles to be added.

As necessary, the production method of the present disclosure may include drying the mixture containing the surface treatment compound and the cellulose derivative particles after the mixing. The drying method is not particularly limited, and known methods such as heat drying, drying under reduced pressure, and vacuum drying may be used. From the viewpoint of high drying efficiency, the drying temperature is preferably room temperature or more, may be 50°C or above, and may be 60°C or above. From the viewpoint of suppressing thermal degradation of the surface treatment compound, the drying temperature is preferably 120°C or below.

Method for Producing Cellulose Derivative Particles

The method for producing the cellulose derivative particles is not particularly limited, and a known method may be used. For example, the cellulose derivative particles may be produced by a production method including a first step of mixing a desired cellulose derivative and a plasticizer to produce a cellulose derivative impregnated with the plasticizer, a second step of kneading the cellulose derivative impregnated with the plasticizer and a water-soluble polymer to produce a dispersion including the cellulose derivative impregnated with the plasticizer as a dispersoid, and a third step of removing the water-soluble polymer from the dispersion to produce cellulose derivative particles. According to this production method, substantially spherical particles having high degree of surface smoothness and sphericity are produced, and thus, the tactile feel of the composition of the present disclosure further improves.

The plasticizer used in the first step is not particularly limited as long as it has a plasticizing effect in melt extrusion processing of the cellulose derivative. Specifically, one of the above-described plasticizers as the plasticizers contained in the cellulose derivative particles may be used alone or two or more thereof may be used in combination. One or two or more selected from the group consisting of acetyl triethyl citrate, triacetin, and diacetin are preferable.

The blending amount of the plasticizer may be greater than 0 parts by weight and less than or equal to 40 parts by weight, from 2 parts by weight to 40 parts by weight, from 10 parts by weight to 30 parts by weight, or from 15 parts by weight to 20 parts by weight per 100 parts by weight of the total amount of the cellulose derivative and the plasticizer. When the blending amount of the plasticizer is within these ranges, particles having higher sphericity can be produced.

The cellulose derivative and the plasticizer may be dry-mixed or wet-mixed using a mixer, such as a Henschel mixer. When a mixer, such as a Henschel mixer, is used, the temperature within the mixer may be a temperature at which the cellulose derivative does not melt, for example, in a range of 20°C or above and lower than 200°C.

The cellulose derivative and the plasticizer may also be mixed by melt-kneading. The melt-kneading may be performed by heating and mixing the cellulose derivative with an extruder. The kneading temperature (cylinder temperature) of the extruder may be from 200°C to 230°C. The melt-kneading performed at a temperature in this range can plasticize the cellulose derivative and provide a uniform kneaded product. The kneaded product may be extruded in a strand shape and formed into a pellet form by hot cutting or the like. The die temperature in this case may be approximately 220°C.

The water-soluble polymer used in the second step refers to a polymer having an insoluble content less than 50 wt.% when 1 g of the polymer is dissolved in 100 g of water at 25°C. As the water-soluble polymer, polyvinyl alcohol, polyethylene glycol, and thermoplastic starch are preferable, and polyvinyl alcohol and thermoplastic starch are more preferable. Further, the thermoplastic starch can be prepared by a well-known method. Specifically, the product produced by mixing tapioca starch with about 20% of glycerin as a plasticizer and kneading the mixture with a twin-screw extruder may be used.

The blending amount of the water-soluble polymer may be from 55 parts by weight to 99 parts by weight per 100 parts by weight of the total amount of the water-soluble polymer and the cellulose derivative impregnated with the plasticizer. The content is preferably from 60 parts by weight to 90 parts by weight, and even more preferably from 65 parts by weight to 85 parts by weight.

In the second step, the water-soluble polymer and the cellulose derivative impregnated with the plasticizer may be kneaded with an extruder, such as a twin-screw extruder. A preferable kneading temperature (cylinder temperature) of the extruder may be from 200°C to 280°C. The dispersion may be extruded in a string shape from a die attached to the tip of an extruder, such as a twin-screw extruder, and then cut into pellets. At this time, the die temperature may be from 220°C to 300°C.

The dispersion produced in the second step is a dispersion containing the water-soluble polymer as a dispersion medium and the cellulose derivative impregnated with the plasticizer as a dispersoid. In other words, the dispersion may have a structure containing the water-soluble polymer as a sea component and the cellulose derivative impregnated with the plasticizer as an island component. In the dispersion, the kneaded product constituting the island component contains the cellulose derivative and the plasticizer and is mainly spherical.

In the third step, the cellulose derivative particles are produced by removing the water-soluble polymer from the dispersion. The method of removing the water-soluble polymer is not particularly limited as long as the water-soluble polymer dissolves and can be removed from the particles. Examples of such methods include a method of dissolving and removing the water-soluble polymer in the dispersion using a solvent, such as water; an alcohol, such as methanol, ethanol, or isopropanol; or a mixed solution of water and such an alcohol. Specific examples include a method of removing the water-soluble polymer from the dispersion by mixing the dispersion and the solvent and then separating the solvent in which the water-soluble polymer is dissolved through a technique such as water-liquid separation through filtration or the like. When the water-soluble polymer is removed by a method of stirring and mixing the dispersion and the solvent and then filtering the mixture, the stirring, mixing, and filtration may be repeated a plurality of times for the purpose of increasing the removal efficiency.

In the third step, the plasticizer may be removed from the dispersion together with the water-soluble polymer but does not have to be removed. Thus, the resulting cellulose derivative particles may contain a plasticizer but does not have to contain a plasticizer.

A mixing ratio of the dispersion and the solvent may be set such that in relation to the total weight of the dispersion and the solvent, the content of the dispersion is from 0.01 wt.% to 20 wt.%, from 2 wt.% to 15 wt.%, or from 4 wt.% to 13 wt.%. When the mixing ratio of the dispersion and the solvent is in this range, efficient washing is possible, and solid-liquid separation after washing is facilitated.

The mixing temperature of the dispersion and the solvent is preferably from 0°C to 200°C, more preferably from 20°C to 110°C, and even more preferably from 40°C to 80°C. When the mixing temperature is within this range, the water-soluble polymer is efficiently removed, and deformation or aggregation of particles due to heating is suppressed. The mixing time of the dispersion and the solvent is appropriately adjusted depending on the mixing temperature and the like.

Method for Producing Cellulose Derivative

The method for producing the cellulose derivative is not particularly limited, and the cellulose derivative may be produced by a known production method.

When the cellulose derivative is a cellulose ester, it can be produced, for example, through activating a raw material pulp (cellulose); acylating the activated cellulose with an esterifying agent (acylating agent); deactivating the acylating agent after the completion of the acylation reaction; and aging (saponifying, hydrolyzing) the produced cellulose acylate. In addition, the method may include, prior to the activation, pretreating the raw material pulp, including disintegrating and grinding the pulp, and then spraying and mixing acetic acid therewith. The method may include post-treating the resulting cellulose acylate, including precipitating and separating, purifying, stabilizing, and drying the cellulose acylate after the aging (saponifying, hydrolyzing).

When the cellulose derivative is a cellulose ether, it can be produced by immersing a raw material pulp (cellulose) in a mixture of a lower aliphatic alcohol, such as isopropyl alcohol (IPA) or tertiary butanol (TBA), water, and an alkali metal hydroxide, such as sodium hydroxide, to produce an alkali cellulose, which is a precursor of the cellulose ether; and further adding an etherifying agent and slurrying (precipitating). Furthermore, the method may include pretreating the raw material pulp to disintegrate/grind it and then to spray and mix acetic acid therewith prior to producing the alkali cellulose. The method may include post-treating the resulting cellulose ether to precipitate and separate, purify, stabilize, and dry it after slurrying (precipitating) the cellulose ether.

Examples

The effects of the present disclosure will be clarified by the examples below, but the present disclosure should not be construed as being limited based on the description of the examples.

Example 1

Zinc stearate (manufactured by NOF Corporation) as a surface treatment compound was charged into a ceramic pot (capacity: 300 mL) containing 400 g of ceramic balls (diameter: 5 mm). Subsequently, cellulose acetate particles (manufactured by DAICEL CORPORATION, total substitution degree = 2.4, average particle size 7.1 $\mu$m) as cellulose

derivative particles were charged into the ceramic pot, and then the ceramic pot was rotated at a rotation speed of 200 rpm for 1 hour using a tabletop pot mill stand (AS ONE Corporation) to produce a composition of Example 1. The amount of zinc stearate charged into the ceramic pot was adjusted so that the content of zinc stearate in the composition was 5 wt.%.

Examples 2 to 26

Compositions of Examples 2 to 26 were produced in the same manner as in Example 1 except that the types and the amounts of the cellulose derivative particles and the surface treatment compound were changed as shown in Table 1 to 4 below. In Examples 4 to 7, 11, 12, 16, 17, 21, and 22, after the surface treatment compound was charged and before the cellulose derivative particles were charged, the ceramic pot was rotated at the rotation speed pf 200 rpm for 1 to 3 hours, and the particles of the surface treatment compound were pulverized until the average particle size of the resulting ground product became 1/3 or less of the average particle size of the cellulose derivative particles. In Examples 3, 10, 15, and 20, the surface treatment compound that had been dried in advance was charged into the ceramic pot. FIGS. 1 and 2 are scanning electron micrographs (3000 times) of the resulting compositions. FIG. 1 is an SEM image of Example 2 in which an organic compound ($N\epsilon$-lauroyl-L-lysine) was used as the surface treatment compound, and FIG. 2 is an SEM image of Example 5 in which an inorganic powder (zinc oxide) was used as the surface treatment compound. In FIG. 2, white fine particles are the inorganic powder.

Comparative Examples 1 to 4

Compositions of Comparative Examples 1 to 4 were obtained in the same manner as in Example 1 except that the surface treatment compound was not used and the cellulose derivative particles were changed to those shown in Table 4 below.

Tactile Feel (Smoothness)

A sensory evaluation of the tactile feel of each Example and the Comparative Example was conducted by a panel of five experts. The experts touched each of the compositions and comprehensively evaluated the compositions in terms of both smoothness and moist feeling on a scale with a maximum score of 5. The average score of the five experts was calculated, and the tactile feel was evaluated according to the following criteria. The evaluation results are shown as tactile feel (smoothness) in Tables 1 to 4 below.
Good: 5. Slightly good: 4. Average: 3. Slightly poor: 2. Poor: 1.

Tactile Feel (Dry Feeling)

A sensory evaluation of the tactile feel of each Example and the Comparative Example was conducted by the panel of five experts. The experts touched each of the compositions and comprehensively evaluated the compositions in terms of dry feeling on a scale with a maximum score of 5. The average score of the five experts was calculated, and the tactile feel was evaluated according to the following criteria. The evaluation results are shown as tactile feel (dry feeling) in Tables 1 to 4 below.
Good: 5. Slightly good: 4. Average: 3. Slightly poor: 2. Poor: 1.

Floatability in Water

The compositions of Examples and Comparative Examples were measured for the floatability in water. Specifically, 1 g of the sample was precisely weighed, added to 50 mL of water, and the mixture was mixed and stirred under the conditions of a rotational speed of 100 rpm or higher and a time of 30 seconds or more. Thereafter, the mixture was allowed to stand for 30 seconds or more, and the particles floating in water were collected and dried, and then the weight thereof was measured. The ratio of the weight of the particles floating in water after drying assuming that the weight of the particles before mixing and stirring with water is 100 is shown in Tables 1 to 4 below as the floatability in water (%). The larger the numerical value, the higher the evaluation.

Floatability in Isododecane

The compositions of Examples and Comparative Examples were measured for the floatability in isododecane. Specifically, 1 g of the sample was precisely weighed, added to 50 mL of isododecane, and the mixture was mixed and stirred under the conditions of a rotational speed of 100 rpm or higher and a time of 30 seconds or more. Thereafter, the mixture was allowed to stand for 30 seconds or more, and the particles floating in isododecane were collected and dried, and then the weight thereof was measured. The ratio of the weight of the particles floating in isododecane after drying assuming that the weight of the particles before mixing and stirring with isododecane is 100 is shown in Tables 1 to 4 below as the floatability in isododecane (%). The smaller the numerical value, the higher the evaluation.

[Table 1]

| | | | Example 1 | Example 2 | Example 3 | Example 4 | Example 5 | Example 6 | Example 7 | Example 8 |
|---|---|---|---|---|---|---|---|---|---|---|
| Cellulose derivative particles | Cellulose derivative | | Cellulose acetate | Cellulose acetate | Cellulose acetate | Cellulose acetate | Cellulose acetate | Cellulose acetate | Cellulose acetate | Cellulose acetate propionate |
| | Total degree of substitution | [-] | 2.4 | 2.4 | 2.4 | 2.4 | 2.4 | 2.4 | 2.4 | 2.58 |
| | Each substitution degree | [-] | Acetyl substitution degree: 2.4 | Acetyl substitution degree: 2.4 | Acetyl substitution degree: 2.4 | Acetyl substitution degree: 2.4 | Acetyl substitution degree: 2.4 | Acetyl substitution degree: 2.4 | Acetyl substitution degree: 2.4 | Acetyl substitution degree: 0.18 Propionyl substitution degree: 2.40 |
| | Average particle size | [$\mu$m] | 7.1 | 7.1 | 7.1 | 7.1 | 7.1 | 7.1 | 7.1 | 9.5 |
| | Coefficient of variation of particle size | (%) | 37 | 37 | 37 | 37 | 37 | 37 | 37 | 34 |
| | Sphericity | [-] | 0.97 | 0.97 | 0.97 | 0.97 | 0.97 | 0.97 | 0.97 | 0.95 |
| | Degree of surface smoothness | (%) | 100 | 100 | 100 | 100 | 100 | 100 | 100 | 100 |
| Surface treatment agent | Surface treatment compound | | Zinc stearate | N$\epsilon$-lauroyl-L-lysine | Stearyltrimethylammonium chloride | Boron nitride | Zinc oxide | Boron nitride | Boron nitride | Zinc stearate |
| | Content | (wt. %) | 5 | 5 | 5 | 5 | 5 | 3 | 10 | 5 |

EP 4 357 403 A1

13

(continued)

| | | Example 1 | Example 2 | Example 3 | Example 4 | Example 5 | Example 6 | Example 7 | Example 8 |
|---|---|---|---|---|---|---|---|---|---|
| Evaluation | Tactile feel (smoothness) [-] | 4.5 | 4.8 | 4.5 | 4.8 | 4.6 | 4.5 | 4.4 | 4.5 |
| | Tactile feel (dry feeling) [-] | 4.8 | 5 | 4.8 | 4.9 | 4.8 | 4.7 | 4.9 | 4.6 |
| | Floatability in water (%) | 100 | 100 | 95 | 100 | 100 | 100 | 100 | 100 |
| | Floatability in isododecane (%) | 0 | 0 | 5 | 0 | 0 | 0 | 0 | 0 |

[Table 2]

| | | | Example 9 | Example 10 | Example 11 | Example 12 | Example 13 | Example 14 | Example 15 | Example 16 |
|---|---|---|---|---|---|---|---|---|---|---|
| Cellulose derivative particles | Cellulose derivative | | Cellulose acetate propionate | Cellulose acetate propionate | Cellulose acetate propionate | Cellulose acetate propionate | Cellulose acetate butylate | Cellulose acetate butylate | Cellulose acetate butylate | Cellulose acetate butylate |
| | Total degree of substitution | [-] | 2.58 | 2.58 | 2.58 | 2.58 | 2.84 | 2.84 | 2.84 | 2.84 |
| | Each substitution degree | [-] | Acetyl substitution degree: 0.18 Propionyl substitution degree: 2.40 | Acetyl substitution degree: 0.18 Propionyl substitution degree: 2.40 | Acetyl substitution degree: 0.18 Propionyl substitution degree: 2.40 | Acetyl substitution degree: 0.18 Propionyl substitution degree: 2.40 | Acetyl substitution degree: 2.13 Butyryl substitution degree: 0.71 | Acetyl substitution degree: 2.13 Butyryl substitution degree: 0.71 | Acetyl substitution degree: 2.13 Butyryl substitution degree: 0.71 | Acetyl substitution degree: 2.13 Butyryl substitution degree: 0.71 |
| | Average particle size | [μm] | 9.5 | 9.5 | 9.5 | 9.5 | 8.2 | 8.2 | 8.2 | 8.2 |
| | Coefficient of variation of particle size | (%) | 34 | 34 | 34 | 34 | 38 | 38 | 38 | 38 |
| | Sphericity | [-] | 0.95 | 0.95 | 0.95 | 0.95 | 0.96 | 0.96 | 0.96 | 0.96 |
| | Degree of surface smoothness | (%) | 100 | 100 | 100 | 100 | 100 | 100 | 100 | 100 |
| Surface treatment agent | Surface treatment compound | | Nε-lauroyl-L-lysine | Stearyltrimethylammonium chloride | Boron nitride | Zinc oxide | Zinc stearate | Nε-lauroyl-L-lysine | Stearyltrimethylammonium chloride | Boron nitride |
| | Content | (wt. %) | 5 | 5 | 5 | 5 | 5 | 5 | 5 | 5 |

EP 4 357 403 A1

(continued)

| Evaluation | | | Example 9 | Example 10 | Example 11 | Example 12 | Example 13 | Example 14 | Example 15 | Example 16 |
|---|---|---|---|---|---|---|---|---|---|---|
| | Tactile feel (smoothness) | [-] | 4.7 | 4.5 | 4.5 | 4.7 | 4.4 | 4.8 | 4.4 | 4.8 |
| | Tactile feel (dry feeling) | [-] | 4.9 | 4.7 | 4.9 | 4.8 | 4.6 | 5 | 4.6 | 4.9 |
| | Floatability in water | (%) | 100 | 95 | 100 | 100 | 100 | 100 | 95 | 100 |
| | Floatability in isododecane | (%) | 0 | 5 | 0 | 0 | 0 | 0 | 5 | 0 |

[Table 3]

| | | | Example 17 | Example 18 | Example 19 | Example 20 | Example 21 | Example 22 |
|---|---|---|---|---|---|---|---|---|
| Cellulose derivative particles | Cellulose derivative | | Cellulose acetate butylate | Ethyl cellulose | Ethyl cellulose | Ethyl cellulose | Ethyl cellulose | Ethyl cellulose |
| | Total degree of substitution | [-] | 2.84 | 2.5 | 2.5 | 2.5 | 2.5 | 2.5 |
| | Each substitution degree | [-] | Acetyl substitution degree: 2.13 Butyryl substitution degree: 0.71 | - | - | - | - | - |
| | Average particle size | [μm] | 8.2 | 7.7 | 7.7 | 7.7 | 7.7 | 7.7 |
| | Coefficient of variation of particle size | (%) | 38 | 38 | 38 | 38 | 38 | 38 |
| | Sphericity | [-] | 0.96 | 0.97 | 0.97 | 0.97 | 0.97 | 0.97 |
| | Degree of surface smoothness | (%) | 100 | 100 | 100 | 100 | 100 | 100 |
| Surface treatment agent | Surface treatment compound | | Zinc oxide | Zinc stearate | Nε-lauroyl-L-lysine | Stearyltrimethylammonium chloride | Boron nitride | Zinc oxide |
| | Content | (wt. %) | 5 | 5 | 5 | 5 | 5 | 5 |
| Evaluation | Tactile feel (smoothness) | [-] | 4.6 | 4.4 | 4.5 | 4.3 | 4.6 | 4.5 |
| | Tactile feel (dry feeling) | [-] | 4.8 | 4.6 | 5 | 4.6 | 4.9 | 4.8 |
| | Floatability in water | (%) | 100 | 100 | 100 | 100 | 100 | 100 |
| | Floatability in isododecane | (%) | 0 | 0 | 0 | 0 | 0 | 0 |

[Table 4]

| | | | Comparative Example 1 | Comparative Example 2 | Comparative Example 3 | Comparative Example 4 | Example 23 | Example 24 | Example 25 | Example 26 |
|---|---|---|---|---|---|---|---|---|---|---|
| Cellulose derivative particles | Cellulose derivative | | Cellulose acetate | Cellulose acetate propionate | Cellulose acetate butylate | Ethyl cellulose | Cellulose acetate | Cellulose acetate | Cellulose acetate | Cellulose acetate |
| | Total degree of substitution | [-] | 2.4 | 2.58 | 2.84 | 2.5 | 2.4 | 2.4 | 2.4 | 2.4 |
| | Each substitution degree | [-] | Acetyl substitution degree: 2.4 | Acetyl substitution degree: 0.18 Propionyl substitution degree: 2.40 | Acetyl substitution degree: 2.13 Butyryl substitution degree: 0.71 | - | Acetyl substitution degree: 2.4 | Acetyl substitution degree: 2.4 | Acetyl substitution degree: 2.4 | Acetyl substitution degree: 2.4 |
| | Average particle size | [μm] | 7.1 | 9.5 | 8.2 | 7.7 | 7.1 | 7.1 | 7.1 | 7.1 |
| | Coefficient of variation of particle size | (%) | 37 | 34 | 38 | 38 | 37 | 37 | 37 | 37 |
| | Sphericity | [-] | 0.97 | 0.95 | 0.96 | 0.97 | 0.97 | 0.97 | 0.97 | 0.97 |
| | Degree of surface smoothness | (%) | 100 | 100 | 100 | 100 | 100 | 100 | 100 | 100 |
| Surface treatment agent | Surface treatment compound | | - | - | - | - | Boron nitride | Boron nitride | Boron nitride | Boron nitride |
| | Content | (wt. %) | - | - | - | - | 1.5 | 20 | 30 | 50 |

EP 4 357 403 A1

18

(continued)

| | | Comparative Example 1 | Comparative Example 2 | Comparative Example 3 | Comparative Example 4 | Example 23 | Example 24 | Example 25 | Example 26 |
|---|---|---|---|---|---|---|---|---|---|
| Evaluation | Tactile feel (smoothness) [-] | 4.1 | 3.8 | 4 | 4.1 | 4.1 | 3.6 | 2.4 | 2 |
| | Tactile feel (dry feeling) [-] | 3.5 | 3.6 | 3.7 | 3.6 | 3.8 | 5 | 5 | 5 |
| | Floatability in water (%) | 0 | 5 | 10 | 5 | 100 | 100 | 100 | 100 |
| | Floatability in isododecane (%) | 100 | 95 | 90 | 95 | 0 | 0 | 0 | 0 |

The details of the compounds shown in Tables 1 to 4 are as follows.

Cellulose acetate particles: manufactured by DAICEL CORPORATION, total substitution degree: 2.4, average particle size: 7.1 μm, sphericity: 0.97, degree of surface smoothness: 100%, weight average molecular weight: 173000

Cellulose acetate propionate particles: manufactured by DAICEL CORPORATION, acetyl substitution degree: 0.18, propionyl substitution degree: 2.40, average particle size: 9.5 μm, sphericity: 0.95, degree of surface smoothness: 100%, weight average molecular weight: 152000

Cellulose acetate butyrate particles: manufactured by DAICEL CORPORATION, acetyl substitution degree: 2.13, butyryl substitution degree: 0.71, average particle size: 8.2 μm, sphericity: 0.96, degree of surface smoothness: 100%, weight average molecular weight: 139000

Ethyl cellulose particles: manufactured by DAICEL CORPORATION, total substitution degree: 2.5, average particle size: 7.7 μm, sphericity: 0.97, degree of surface smoothness: 100%, weight average molecular weight: 182000

Zinc stearate: manufactured by NOF Corporation

Nε-lauroyl-L-lysine: trade name "AMIHOPE LL" manufactured by AJINOMOTO CO., INC.

Stearyltrimethylammonium chloride: trade name "CA-2450" manufactured by Nikko Chemicals Co., Ltd.

Boron nitride: trade name "SHP-3" manufactured by Mizushima Ferroalloy Co., Ltd.

Zinc oxide: trade name "FINEX-50" manufactured by Sakai Chemical Industry Co., Ltd.

Evaluation of Sebum Solidifying Effect

Artificial sebum was added to each of the compositions of Example 12 and Comparative Example 2 at a weight ratio of 1 : 1, followed by stirring and mixing. The sample was allowed to stand at 40°C, and the state after a predetermined time was visually observed. The formulation of the artificial sebum was 35 wt.% of olive oil, 13 wt.% of oleic acid, 25 wt.% of jojoba oil, 15 wt.% of squalene, 7 wt.% of "ELDEW PS-203" (trade name), and 5 wt.% of glyceryl tri-2-ethylhexanoate.

As a result of the observation over time, it was confirmed that the composition of Example 12 started to be solidified after 0.5 hour and was completely solidified after 1 hour. On the other hand, in Comparative Example 2, the fluidity was the same as that at the start of the test even after 2 hours.

As shown in Tables 1 to 4, the compositions of Examples were highly rated for tactile feel in comparison to the compositions of Comparative Examples. Since all the compositions of Examples floated in water and settled in isodecane, it is found that the compositions are excellent in affinity with a hydrophobic solvent. Further, it is found that the composition containing zinc oxide as the surface treatment compound provides a good sebum solidifying effect. From this evaluation result, the superiority of the present disclosure is clear.

Industrial Applicability

The composition described above may be applied to the production of various cosmetic compositions.

**Claims**

1. A composition comprising:

   particles of a cellulose derivative; and
   a surface treatment compound,
   wherein
   the particles of the cellulose derivative have an average particle size from 0.08 μm to 100 μm,
   the surface treatment compound is an organic compound having a long-chain alkyl group and/or an inorganic powder, and
   the inorganic powder has an average particle size of 1/3 or less of the average particle size of the particles of the cellulose derivative.

2. The composition according to claim 1, wherein the long-chain alkyl group has from 8 to 22 carbon atoms.

3. The composition according to claim 1 or 2, wherein the organic compound is a compound selected from the group consisting of a cationic surfactant and a metal soap, or Nε-lauroyl-L-lysine.

4. The composition according to any one of claims 1 to 3, wherein the surface treatment compound is one or two or more selected from the group consisting of zinc stearate, Nε-lauroyl-L-lysine, boron nitride, and zinc oxide.

5. The composition according to any one of claims 1 to 4, wherein a content of the surface treatment compound is from 3.0 wt.% to 10.0 wt.%.

6. The composition according to any one of claims 1 to 5, wherein the cellulose derivative has an alkoxyl group having from 2 to 20 carbon atoms or an acyl group having from 2 to 40 carbon atoms.

7. The composition according to any one of claims 1 to 6, wherein the cellulose derivative is cellulose acylate.

8. The composition according to any one of claims 1 to 7, wherein the cellulose derivative is cellulose acetate.

9. The composition according to any one of claims 1 to 8, wherein a total substitution degree of the cellulose derivative is from 2.0 to 3.2.

10. The composition according to any one of claims 1 to 9, wherein the particles of the cellulose derivative have a sphericity from 0.7 to 1.0 and a degree of surface smoothness from 80% to 100%.

11. A cosmetic composition comprising the composition described in any one of claims 1 to 10.

12. A method for producing the composition described in any one of claims 1 to 10, the method comprising:

mixing particles of a cellulose derivative having an average particle size from 0.08 μm to 100 μm and a surface treatment compound, wherein the particles of the cellulose derivative and the surface treatment compound are dry-mixed,
wherein
the surface treatment compound is an organic compound having a long-chain alkyl group and/or an inorganic powder, and
the inorganic powder has an average particle size of 1/3 or less of the average particle size of the particles of the cellulose derivative.

13. The method for producing the composition according to claim 12, the method further comprising pulverizing the surface treatment compound before the mixing.

2020/09/29     H L     x3.0k     30 um

# FIG. 1

2020/09/29     H L     x3.0k     30 um

# FIG. 2

FIG. 3

FIG. 4

## INTERNATIONAL SEARCH REPORT

| International application No. |
| --- |
| **PCT/JP2022/018738** |

**A. CLASSIFICATION OF SUBJECT MATTER**

*C08L 1/02*(2006.01)i; *A61K 8/73*(2006.01)i; *A61Q 1/00*(2006.01)i; *C08K 3/22*(2006.01)i; *C08K 3/38*(2006.01)i; *C08K 5/098*(2006.01)i; *C08L 1/12*(2006.01)i
FI:  C08L1/02; A61Q1/00; C08L1/12; C08K3/22; A61K8/73; C08K3/38; C08K5/098

According to International Patent Classification (IPC) or to both national classification and IPC

**B. FIELDS SEARCHED**

Minimum documentation searched (classification system followed by classification symbols)

C08L1/02; A61K8/73; A61Q1/00; C08K3/22; C08K3/38; C08K5/098; C08L1/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | WO 2020/188698 A1 (DAICEL CORPORATION) 24 September 2020 (2020-09-24) claims, paragraphs [0019], [0055], [0072], [0086], [0092], [0101]-[0103], [0125], each example | 1-13 |
| Y | | 6-9 |
| X | JP 2020-075878 A (MATSUMOTO YUSHI SEIYAKU KK) 21 May 2020 (2020-05-21) claims, paragraphs [0004], [0023], [0031], [0041], examples 1-2 | 1-5, 10-13 |
| Y | | 6-9 |
| X | JP 2021-066704 A (MATSUMOTO YUSHI SEIYAKU KK) 30 April 2021 (2021-04-30) claims, paragraphs [0004], [0012], [0018], [0020], [0031], [0038] | 1-5, 10-11 |
| Y | | 6-9 |
| A | | 12-13 |
| A | JP 2018-118956 A (AJINOMOTO KK) 02 August 2018 (2018-08-02) | 1-13 |
| A | WO 2019/156116 A1 (DAICEL CORPORATION) 15 August 2019 (2019-08-15) | 1-13 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **01 July 2022** | **12 July 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2022/018738**

**C. DOCUMENTS CONSIDERED TO BE RELEVANT**

| Category* | Citation of document, with indication, where appropriate, of the relevant passages | Relevant to claim No. |
|---|---|---|
| A | JP 2020-152851 A (DAICEL CORPORATION) 24 September 2020 (2020-09-24) | 1-13 |

Form PCT/ISA/210 (second sheet) (January 2015)

25

**INTERNATIONAL SEARCH REPORT**
Information on patent family members

International application No.

**PCT/JP2022/018738**

| Patent document cited in search report | | | Publication date (day/month/year) | Patent family member(s) | | | Publication date (day/month/year) |
|---|---|---|---|---|---|---|---|
| WO | 2020/188698 | A1 | 24 September 2020 | JP | 6694559 | B1 | |
| | | | | KR | 10-2021-0127741 | A | |
| | | | | CN | 113710729 | A | |
| | | | | TW | 202034896 | A | |
| JP | 2020-075878 | A | 21 May 2020 | (Family: none) | | | |
| JP | 2021-066704 | A | 30 April 2021 | (Family: none) | | | |
| JP | 2018-118956 | A | 02 August 2018 | WO | 2018/062315 | A1 | |
| WO | 2019/156116 | A1 | 15 August 2019 | US | 2020/0179261 | A1 | |
| | | | | EP | 3613794 | A1 | |
| | | | | CN | 110650994 | A | |
| | | | | KR | 10-2019-0135537 | A | |
| JP | 2020-152851 | A | 24 September 2020 | US | 2020/0299488 | A1 | |
| | | | | EP | 3711747 | A1 | |
| | | | | KR | 10-2020-0112641 | A | |
| | | | | CN | 111718426 | A | |
| | | | | TW | 202100565 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6609726 B
- JP 2020152851 A

- JP 6694559 B

**Non-patent literature cited in the description**

- **TEZUKA.** *Carbonydr. Res.,* 1995, vol. 273, 83